# EUROPÄISCHE PATENTSCHRIFT

(11) **EP 2 594 191 B1**
(45) Veröffentlichungstag und Bekanntmachung des Hinweises auf die Patenterteilung: **05.04.2017**
(21) Anmeldenummer: 12192273.6
(22) Anmeldetag: 12.11.2012
(51) Int. Cl.: A61B 1/06, F21L 14/00, G02B 3/14, G02B 23/24

(54) **Beleuchtungsoptik und Beobachtungseinrichtung zum Erzeugen eines Beleuchtungslichtbündels**
Illumination lens and observation device for generating an illumination light bundle
Optique d'éclairage et installation d'observation de génération d'un faisceau lumineux d'éclairage

(30) Priorität: 15.11.2011 DE 102011086325
(43) Veröffentlichungstag der Anmeldung: 22.05.2013
(73) Patentinhaber: Karl Storz GmbH & Co. KG, 78532 Tuttlingen (DE)
(72) Erfinder: Dahmen, Jan, 78606 Seitingen (DE)

(56) Entgegenhaltungen:
- EP-A2- 1 747 751
- WO-A2-2007/033326
- WO-A2-2007/051173
- DE-U1-202007 004 527
- US-A1- 2009 177 033
- US-A1- 2010 142 059

## Beschreibung

Die vorliegende Erfindung betrifft eine Beleuchtungsoptik, insbesondere für eine medizinische Stirnlampe, ein Endoskop oder ein Exoskop, sowie eine Beobachtungseinrichtung, insbesondere eine medizinische Stirnlampe, ein Endoskop oder ein Exoskop, und weiterhin ein Verfahren zum Erzeugen eines Beleuchtungslichtbündels mit veränderbarer Lichtverteilung.

Medizinische Stirnlampen umfassen ein Tragegestell oder einen Stirnreif, mit dem eine Beleuchtungseinheit auf dem Kopf eines Benutzers getragen werden kann, sowie die Beleuchtungseinheit selbst, die oberhalb der Augen des Benutzers getragen wird. Die Beleuchtungseinheit kann eine Lichtquelle umfassen oder einen Lichtleiter, durch den das von einer externen Lichtquelle erzeugte Licht in die Beleuchtungseinheit übertragen wird. Die Beleuchtungseinheit weist eine Beleuchtungsoptik auf, durch die das von der Lichtquelle erzeugte bzw. durch den Lichtleiter weitergeleitete Beleuchtungslicht auf ein Objekt, etwa auf eine Gewebeoberfläche, projiziert wird, um ein für eine medizinische Untersuchung oder für einen chirurgischen Eingriff benötigtes Objektfeld auszuleuchten. Eine medizinische Stirnlampe kann auch mit einer Sehhilfe, etwa einer Binokularlupe, oder auch mit einer kopfgetragenen Videokamera versehen sein.

Bei einer medizinischen Untersuchung oder einem chirurgischen Eingriff ist es wünschenswert, dass das Untersuchungs- bzw. Operationsgebiet mit einer hohen, zur Erkennung aller Details ausreichenden Lichtstärke möglichst homogen ausgeleuchtet wird. Der beleuchtete Objektbereich soll möglichst scharfrandig begrenzt sein, so dass möglichst wenig Beleuchtungslicht durch Ausleuchtung von außerhalb des Untersuchungs- bzw. Operationsgebiets liegenden Bereichen verloren geht; ferner wird durch eine scharfrandige Begrenzung des ausgeleuchteten Objektbereichs die gesamte Wärmeentwicklung sowie die Gefahr der Blendung anderer Personen reduziert.

Weiterhin kann es erforderlich sein, unterschiedlich große Objektbereiche und/oder Objektbereiche in unterschiedlichen Arbeitsabständen auszuleuchten. Wenn die Beleuchtungsoptik ein Beleuchtungslichtbündel mit einem großen Öffnungswinkel erzeugt, so ist ein großer Objektbereich ausleuchtbar. Andererseits kann die Lichtstärke dann für das Erkennen von Details unzureichend sein, insbesondere bei einem relativ großen Arbeitsabstand. Ist andererseits der Öffnungswinkel klein, so kann zwar auch in einem größeren Arbeitsabstand noch eine ausreichende Beleuchtungsstärke erzielt werden. Insbesondere bei kleinem Arbeitsabstand kann der ausgeleuchtete Objektbereich für die vorgesehene Untersuchung bzw. Operation jedoch zu klein sein. Auch in unterschiedlichen Arbeitsabständen ist jeweils eine scharfrandige Begrenzung des ausgeleuchteten Objektbereichs wünschenswert.

Es sind deshalb Stirnlampen entwickelt worden, die eine Veränderung der Lichtverteilung eines erzeugten Beleuchtungslichtbündels ermöglichen.

Gemäß US 5,430,620 ist der Austrittsfläche eines Lichtleiters eine Linse vorgelagert, die über ein Schraubgewinde entlang der optischen Achse des Endbereichs des Lichtleiters verschoben werden kann, so dass der Abstand zwischen der Linse und der Ausgangsfläche des Lichtleiters verändert werden kann. Dies ermöglicht eine Anpassung der Größe und der Helligkeit des ausgeleuchteten Objektbereichs an die jeweiligen Erfordernisse, wobei nur in der Position des größten Abstands der Linse von der Lichtleiterendfläche die Austrittsfläche des Lichtleiters scharf abgebildet wird. In US 7,314,300 B1 ist eine mehrlinsige Beleuchtungsoptik einer chirurgischen Stirnlampe offenbart, bei der der Abstand zwischen einer Lichtleiterendfläche und einer Konvexlinse durch Verschieben eines Lichtleiteransatzes verändert werden kann. Hierdurch kann die Lichtverteilung in einem ausgeleuchteten Objektbereich verändert werden. Eine Anpassung an unterschiedliche Arbeitsabstände ist hierbei ebenfalls nicht vorgesehen.

Aus DE 32 11 187 A1 ist es bekannt, einen auf ein freies Ende eines faseroptischen Lichtleiters aufgesetzten Glasstab zusammen mit einer seiner lichtabstrahlenden Fläche unmittelbar benachbarten Blende gegenüber einem Kollektor axial zu verschieben, um die Blende in verschiedene Arbeitsabständen scharf abzubilden. Hierdurch kann ein in einer vorwählbaren Ebene liegendes Beobachtungsfeld jeweils randscharf ausgeleuchtet werden. Der Kollektor kann dabei als Vario-System ausgebildet sein; ein solches System umfasst eine Mehrzahl von Linsen, die relativ zueinander verschoben werden und ist mit einem hohen Fertigungs- und Justageaufwand verbunden.

Die aus dem Stand der Technik bekannte Veränderung der Lichtverteilung eines Beleuchtungslichtbündels durch mechanische Verstellung einer oder mehrerer Linsen bzw. eines Lichtleiterendes verursacht aufgrund der im medizinischen Bereich geltenden Reinigungs- und Sterilisierungsanforderungen einen erhöhten Abdichtungsaufwand; dies gilt insbesondere dann, wenn die Beleuchtungsoptik Teil einer medizinischen Stirnlampe ist oder im distalen Endbereich eines Endoskops oder eines Exoskops angeordnet ist. Unter "Exoskop" wird eine Beobachtungseinrichtung verstanden, die für einen Einsatz außerhalb eines Hohlraums bestimmt ist, wie beispielsweise in DE 10 2011 001 200 A1 beschrieben. Sowohl ein Endoskop als auch ein Exoskop kann eine Beleuchtungsoptik zur Erzeugung eines Beleuchtungslichtbündels umfassen bzw. einem Endoskop oder einem Exoskop kann eine separate Beleuchtungseinheit mit einer derartigen Beleuchtungsoptik zugeordnet sein.

Die genannten Lösungen zur Veränderung der Lichtverteilung führen auch zu einer Vergrößerung der Baugröße und des Gewichts der Beleuchtungseinheit, was ebenfalls unerwünscht ist. Beleuchtungsoptiken, bei denen lediglich eine Linse verschoben werden kann oder ein Lichtleiterende relativ zu einer feststehenden Linsenanordnung verschoben werden kann, ermöglichen keine unabhängige Einstellung einer Fokussierung und einer Größe eines ausgeleuchteten Objektbereichs in unterschiedlichen Arbeitsabständen.

DE 20 2007 004527 U1 zeigt eine Flüssiglinse zur Verwendung insbesondere für eine Taschenlampe, die zur Fokussierung dient und dazu ausschließlich elektrisch steuerbar ist.

In EP 1 747 751 A2 ist eine Fluidlinse für ein optisches medizinisches Instrument gezeigt, die ebenso über elektrische Ansteuerung fokussierbar ist.

Aus US 2009/0177033 A1 geht eine Fluidlinse für eine medizinische Diagnose-Kapsel hervor, wiederum elektrisch ansteuerbar.

Ebenso für medizintechnische Vorrichtungen schlägt WO 2007/033326 A2 Fluidlinsensysteme vor, das wiederum über variable Spannung bzgl. Fokussierung steuerbar ist.

In der Druckschrift US 2010/0142059, die alle Merkmale des Oberbegriffs des Anspruchs 1 zeigt, ist eine Flüssiglinse mit zwei Flüssigkeiten gezeigt, die durch eine Membran getrennt und über eine Leitung, die aus dem Linsenraum heraus eine Bypass bildet, in welchem die beiden Flüssigkeiten ebenso getrennt sind, und zwar durch eine Trennschicht, die von außerhalb der Bypass-Leitung ansteuerbar ist, so dass die Trennschicht die Volumina der beiden Flüssigkeiten zueinander verändern kann, in der Bypass-Leitung wie gegengleich im Linsenraum.

Es ist Aufgabe der vorliegenden Erfindung, eine Beleuchtungsoptik, insbesondere für eine medizinische Stirnlampe, ein Endoskop oder ein Exoskop, zum Erzeugen eines Beleuchtungslichtbündels mit veränderbarer Lichtverteilung, sowie eine Beobachtungseinrichtung, insbesondere eine medizinische Stirnlampe, ein Endoskop oder ein Exoskop, und weiterhin ein Verfahren zum Erzeugen eines Beleuchtungslichtbündels mit veränderbarer Lichtverteilung anzugeben, wobei die unabhängige Einstellung einer Fokussierung und einer Größe eines ausgeleuchteten Objektbereichs in unterschiedlichen Arbeitsabständen auf einfache Art realisiert werden.

Diese Aufgabe wird durch eine Beleuchtungsoptik gemäß Anspruch 1 gelöst.

Vorteilhafte Weiterbildungen der Erfindung ergeben sich aus den Unteransprüchen.

Die Erfindung betrifft eine Beleuchtungsoptik für eine medizinische Stirnlampe, ein insbesondere medizinisches Endoskop oder ein insbesondere medizinisches Exoskop. Eine derartige medizinische Stirnlampe kann auch eine Beobachtungseinrichtung, insbesondere eine Videokamera, umfassen. Ein derartiges Endoskop oder Exoskop, insbesondere ein medizinisches Endoskop oder Exoskop, kann eine erfindungsgemäße Beleuchtungsoptik umfassen; die Beleuchtungsoptik kann aber auch Teil einer dem Endoskop bzw. Exoskop zugeordneten, jedoch von diesem separaten Beleuchtungseinheit sein.

Die erfindungsgemäße Beleuchtungsoptik ist zum Erzeugen eines Beleuchtungslichtbündels ausgebildet. Insbesondere ist die Beleuchtungsoptik zum Ausleuchten eines Objektbereichs durch Projizieren des von einer Lichtquelle erzeugten Beleuchtungslichts auf den Objektbereich ausgebildet. Die Lichtquelle kann Teil der Stirnlampe bzw. des Endoskops oder Exoskops sein, kann aber auch eine hiervon separate Einheit darstellen, wobei das von der Lichtquelle erzeugte Beleuchtungslicht über einen Lichtleiter, beispielsweise ein Bündel von Lichtleitfasern, zur Beleuchtungsoptik übertragen werden kann. Um einen möglichst großen Anteil des von der Lichtquelle erzeugten Lichts zu sammeln und zur Beleuchtungsoptik zu leiten, kann etwa eine Einkopplungsoptik zur Einkopplung des von der Lichtquelle erzeugten Lichts in den Lichtleiter vorgesehen sein oder eine mit einer Lichtquelle verbundene Kollimatoroptik. Die Lichtquelle kann beispielsweise eine Xenon-Lampe oder eine lichtemittierende Diode (LED) bzw. eine Anordnung von LEDs sein. Die Beleuchtungsoptik kann zum Erzeugen des Beleuchtungslichtbündels eine Linsenanordnung aufweisen, die eine oder mehrere Linsen sowie ggf. weitere andere optische Elemente, wie beispielsweise Spiegel und/oder Blenden, umfassen kann.

Die erfindungsgemäße Beleuchtungsoptik ist zum Erzeugen eines Beleuchtungslichtbündels mit veränderbarer Lichtverteilung ausgebildet. Insbesondere kann der Öffnungswinkel des Beleuchtungslichtbündels, der auch als Beleuchtungswinkel bezeichnet wird, und/oder der Lichtstärkeabfall im Randbereich des Beleuchtungslichtbündels veränderbar sein. Es können auch weitere Eigenschaften der Lichtverteilung, etwa die Homogenität der Ausleuchtung eines Objektfelds, veränderbar sein. Die Helligkeitsverteilung innerhalb eines ausgeleuchteten Objektfelds kann vom Abstand des Objektfelds von der Beleuchtungsoptik abhängen.

Erfindungsgemäß weist die Beleuchtungsoptik eine Flüssiglinse (Liquidlinse) auf, deren Brennweite zur Veränderung einer Fokussierung des Beleuchtungslichtbündels veränderbar ist. Durch Veränderung der Brennweite der Flüssiglinse ist somit eine Konvergenz bzw. Divergenz von Strahlen des Beleuchtungslichtbündels veränderbar. Insbesondere kann die Konvergenz bzw. Divergenz von Strahlen, die von einem Punkt einer Lichtquelle oder einer Endfläche eines Lichtleiters ausgehen, verändert werden. Ferner können hierdurch weitere Eigenschaften der Lichtverteilung, etwa der Öffnungswinkel des Beleuchtungslichtbündels, veränderbar sein. Die Beleuchtungsoptik kann eine Linsenanordnung aufweisen, die die Flüssiglinse mit veränderbarer Brennweite umfasst.

Dadurch, dass die Beleuchtungsoptik eine Flüssiglinse mit veränderbarer Brennweite zur Veränderung einer Fokussierung des Lichtbündels aufweist, kann auf einfache Weise eine Veränderung der Lichtverteilung des von der Beleuchtungsoptik erzeugen Beleuchtungslichtbündels erreicht werden. Insbesondere dann, wenn die Beleuchtungsoptik kein weiteres abbildendes optisches Element aufweist, insbesondere keine weitere Linse als die Flüssiglinse mit veränderbarer Brennweite, ist ein konstruktiv besonders einfacher Aufbau der Beleuchtungsoptik erreichbar. Hierdurch wird auch eine Ausführung der Beleuchtungsoptik mit einem besonders geringen Bauraum und einem besonders geringen Gewicht ermöglicht. Ferner ist zur Veränderung der Brennweite der Flüssiglinse in der Regel keine mechanische Verschiebung eines optischen Elements erforderlich, wodurch die Abdichtung der Beleuchtungsoptik bzw. einer Beleuchtungseinheit, die die Beleuchtungsoptik umfasst, vereinfacht wird.

Gemäß einer bevorzugten Ausführungsform der Erfindung ist die Brennweite der Flüssiglinse elektrisch verstellbar. Insbesondere kann die Brennweite der Flüssiglinse durch eine elektrische Spannung veränderbar sein, die ein elektrisches Feld erzeugt, das über den Effekt der Elektrobenetzung den Kontaktwinkel einer in der Flüssiglinse enthaltenen Flüssigkeit und damit die Krümmung der optisch wirksamen Grenzfläche steuert. Die Einstellung der elektrischen Spannung kann beispielsweise durch eine Taste oder ein anderes Bedienelement erfolgen, das der Beleuchtungseinheit zugeordnet oder an dieser angeordnet ist. Dadurch, dass die Brennweite der Flüssiglinse elektrisch veränderbar ist, wird eine besonders vielseitige Ansteuerung der Flüssiglinse zur Veränderung der Lichtverteilung des Beleuchtungslichtbündels ermöglicht. Ferner wird eine Konstruktion ohne mechanisch bewegliche Elemente und dadurch ein besonders einfacher Aufbau der Beleuchtungseinheit ermöglicht, wobei keine zusätzlichen Maßnahmen zur Abdichtung notwendig sind.

Alternativ oder zusätzlich kann eine Einstellung der Brennweite der Flüssiglinse durch mechanische Mittel erfolgen. So kann die Flüssiglinse beispielsweise eine Fassung aufweisen, in der die eine optisch wirksame Grenzfläche bildenden Flüssigkeiten aufgenommen sind und deren Radius mechanisch veränderbar ist. Vorzugsweise umfasst die Beleuchtungsoptik bzw. die Beleuchtungseinheit ein mechanisches Bedienelement, insbesondere ein Drehrad, das direkt oder beispielsweise über ein Getriebe auf die Flüssiglinse zur Veränderung der Brennweite einwirkt. Hierdurch wird mit einem einfach bedienbaren Bedienelement und mit einem vergleichsweise einfachen Aufbau der Beleuchtungsoptik eine Veränderung der Lichtverteilung des Beleuchtungslichtbündels ermöglicht, wobei die Abdichtung nur einen relativ geringen Aufwand erfordert.

Weiterhin ist es bevorzugt, dass die Beleuchtungsoptik zum randscharfen Ausleuchten eines Objektbereichs in unterschiedlichen Arbeitsabständen durch Einstellung unterschiedlicher Brennweiten der Flüssiglinse ausgebildet ist. Dadurch, dass die Linsenanordnung eine Flüssiglinse mit veränderbarer Brennweite umfasst, wird eine scharfrandige Ausleuchtung eines Objektfelds in unterschiedlichen Arbeitsabständen mit einem besonders einfachen optischen und mechanischen Aufbau der Beleuchtungsoptik ermöglicht. Dies gilt insbesondere in dem Fall, dass die Linsenanordnung außer der Flüssiglinse keine weiteren Linsen aufweist.

Insbesondere ist die Beleuchtungsoptik zur Abbildung einer Lichtaustrittsebene auf den Objektbereich ausgebildet. Beispielsweise kann die Flüssiglinse derart angeordnet sein, dass die Lichtaustrittsebene mit einer proximalseitigen Brennebene zusammenfällt, die einer Brennweite in einer ersten Einstellung der Flüssiglinse entspricht, wobei zur Abbildung der Lichtaustrittsebene auf den Objektbereich die Brennweite gegenüber der ersten Einstellung verringert werden kann. Eine scharfe Abbildung der Lichtaustrittsebene auf den Objektbereich ermöglicht insbesondere eine scharfe Begrenzung der ausgeleuchteten Fläche. Durch die Abbildung der Lichtaustrittsebene auf den Objektbereich kann dieser besonders homogen und scharfrandig ausgeleuchtet werden.

Die Lichtaustrittsebene kann insbesondere die Ebene einer Blende, einer Austrittsfläche eines Lichtleiters oder auch einer Streuscheibe sein. Insbesondere dann, wenn die Lichtquelle Teil der medizinischen Stirnlampe bzw. des Endoskops oder des Exoskops ist, stellt eine Blende eine vorteilhafte Möglichkeit zur Begrenzung des ausgeleuchteten Objektbereichs dar. Zur weiteren Verbesserung der Homogenität der Ausleuchtung des Objektbereichs kann auch eine Streuscheibe in der Lichtaustrittsebene vorgesehen sein. Die Blende bzw. Streuscheibe kann Teil der Beleuchtungsoptik sein. In dem Fall, dass die Lichtquelle separat angeordnet ist und das Beleuchtungslicht über einen Lichtleiter zur Beleuchtungsoptik übertragen wird, ist die Lichteintrittsebene insbesondere die Endfläche des Lichtleiters. Diese kann eine ebene Fläche sein, in der die Endflächen der Lichtleitfasern angeordnet sind.

Während prinzipiell eine Abbildung der Lichtaustrittsebene auf den Objektbereich zur Erzeugung einer scharfen Begrenzung des ausgeleuchteten Bereichs wünschenswert ist, ist eine geringfügig defokussierte Abbildung der Lichtaustrittsebene besonders vorteilhaft. Hierdurch kann beispielsweise bei der Abbildung einer Lichtleiterendfläche auf den Objektbereich verhindert werden, dass eine inhomogene Ausleuchtung der Lichtleiterendfläche aufgrund der dunklen Zwischenräume zwischen den Lichtleitfasern zu einer inhomogenen Ausleuchtung des Objektbereichs führt. Ferner kann auf diese Weise praktisch ohne Lichtverluste eine homogene Ausleuchtung des Objektbereichs erreicht werden. Die Randschärfe des ausgeleuchteten Objektbereichs wird hierdurch nur unwesentlich oder überhaupt für den Benutzer nicht feststellbar verringert. Wenn in der vorliegenden Anmeldung von "scharf" oder "randscharf" die Rede ist, ist daher eine derartige nahezu scharfe, d.h. geringfügig defokussierte, Ausleuchtung bzw. Abbildung mit umfasst.

Gemäß einer bevorzugten Ausführungsform der Erfindung ist die Beleuchtungsoptik zum Verändern eines Öffnungswinkels des Beleuchtungslichtbündels ausgebildet. Das Beleuchtungslicht ist somit mit unterschiedlichen Beleuchtungswinkeln auf den Objektbereich projizierbar. Das Beleuchtungslicht wird beispielsweise in Form eines Lichtkegels auf den Objektbereich projiziert, wobei der Beleuchtungswinkel den Öffnungswinkels des Lichtbündels bzw. den Winkeldurchmesser des Kegels angibt. Der Beleuchtungswinkel kann in unterschiedlichen Richtungen unterschiedlich groß sein; im Folgenden wird auch in diesem Fall vereinfachend von "dem Beleuchtungswinkel" gesprochen. Auf diese Weise kann es ermöglicht werden, dass unabhängig vom Abstand des Objektbereichs von der Beleuchtungsoptik ein in einer gewünschten Größe einstellbarer Objektbereich ausgeleuchtet werden kann.

In bevorzugter Weise ist die Flüssiglinse entlang einer optischen Achse der Beleuchtungsoptik bewegbar, insbesondere verschiebbar, gelagert. Zur Bewegung bzw. Verschiebung der Flüssiglinse kann beispielsweise ein mechanischer oder elektromotorischer Antrieb vorgesehen sein. Durch Verschiebung der Flüssiglinse entlang der optischen Achse und durch Einstellung der Brennweite der Flüssiglinse wird es auf besonders einfache Weise ermöglicht, bei unterschiedlichen Abständen des Objektbereichs von der Beleuchtungsoptik einen Objektbereich einer jeweils gewünschten Größe auszuleuchten. Insbesondere ist hierfür keine weitere Linse als die Flüssiglinse erforderlich; es kann aber auch eine Linsenanordnung vorgesehen sein, die weitere Linsen oder optisch wirksame Flächen umfassen kann.

In besonders bevorzugter Weise sind die Einstellung der Brennweite der Flüssiglinse und die Verschiebung der Flüssiglinse entlang der optischen Achse der Linsenanordnung miteinander derart gekoppelt, dass bei einem festen Arbeitsabstand Objektbereiche unterschiedlicher Größe jeweils mit einer scharfen Begrenzung ausgeleuchtet werden können. Es kann aber auch beispielsweise vorgesehen sein, dass die Einstellung der Brennweite und die Verschiebung der Flüssiglinse derart miteinander gekoppelt sind, dass ein Objektbereich vorgebbarer Größe in unterschiedlichen Abständen zur Beleuchtungsoptik jeweils mit einer scharfen Begrenzung ausgeleuchtet wird. Auf diese Weise wird eine besonders einfache Bedienung der Beleuchtungsoptik in jeweils wichtigen Anwendungsbereichen ermöglicht.

Insbesondere in dem Fall, dass die Brennweite der Flüssiglinse durch mechanische Mittel veränderbar ist, ist die mit der Einstellung der Brennweite gekoppelte Bewegung der Flüssiglinse entlang der optischen Achse vorzugsweise mechanisch mit einem mechanischen Bedienelement gekoppelt. So kann beispielsweise eine Verschiebung der Flüssiglinse über einen Gewindetrieb mit der Drehung eines Drehrads gekoppelt sein, das zur Veränderung der Brennweite der Flüssiglinse betätigbar ist. Sofern die Brennweite der Flüssiglinse elektrisch verstellbar ist, kann eine Verschiebung der Flüssiglinse entlang der optischen Achse der Beleuchtungsoptik ebenfalls elektrisch, insbesondere elektromotorisch, betätigbar sein. In besonders vorteilhafter Weise kann die Beleuchtungsoptik sowohl zur Einstellung der Brennweite der Flüssiglinse als auch zur Verstellung der Flüssiglinse entlang der optischen Achse mit einer elektronischen Steuerung verbindbar sein. Hierdurch wird in besonders vielseitig einsetzbarer und komfortabler Weise eine Veränderung der Brennweite der Flüssiglinse, eine Verschiebung entlang der optischen Achse und eine Kopplung beider Verstellmöglichkeiten ermöglicht.

Eine erfindungsgemäße Beobachtungseinrichtung, die vorzugsweise eine medizinische Beobachtungseinrichtung ist, insbesondere eine medizinische Stirnlampe mit einer Kameraeinrichtung, ein Endoskop oder ein Exoskop, weist eine Beleuchtungsoptik der oben beschriebenen Art auf. Dabei kann die Beleuchtungsoptik in die Beobachtungseinrichtung integriert sein oder dieser als Teil einer separaten Beleuchtungseinheit zugeordnet sein. Die Beobachtungseinrichtung weist ferner eine Beobachtungsoptik auf, die etwa ein Kameraobjektiv oder eine Objektiv mit einem Bildweiterleiter, beispielsweise einem Relaislinsensystem oder einem Bildleiter, sein kann. Hierdurch wird eine Beobachtungseinrichtung, insbesondere eine medizinische Stirnlampe mit Beobachtungseinrichtung bzw. ein Endoskop oder Exoskop, geschaffen, bei dem auf einfache Weise die Lichtverteilung eines Beleuchtungslichtbündels veränderbar ist. Hierdurch ist insbesondere ein randscharfes Ausleuchten eines Objektbereichs in unterschiedlichen Arbeitsabständen durch Anpassung der Brennweite der Flüssiglinse möglich.

Gemäß einer bevorzugten Ausführungsform weist die Beobachtungeinrichtung eine Beobachtungsoptik mit einem veränderbaren Sichtwinkel auf, beispielsweise eine Zoomoptik einer Videokamera, und die Beleuchtungsoptik ist zur Anpassung eines Beleuchtungswinkels an den veränderbaren Sichtwinkel ausgebildet. Die Beobachtungseinrichtung kann etwa eine Stirnlampe mit einer Kameraeinrichtung mit einer Zoomoptik oder eine Sehhilfe mit veränderlicher Vergrößerung sein; auch bei Endoskopen oder Exoskopen ist es bekannt, das Sichtfeld durch eine Zoomoptik zu verändern. Hierdurch wird es auf einfache und wirksame Weise ermöglicht, bei unterschiedlichen Sichtwinkeln stets eine optimale, insbesondere stets eine vollständige und ausreichend helle Ausleuchtung eines Objektfelds zu wählen. Dadurch ist insbesondere der randscharf ausleuchtbare Objektbereich an eine gewählte Vergrößerung der Beobachtungseinrichtung anpassbar.

Gemäß einer bevorzugten Ausführungsform der Erfindung ist die Flüssiglinse zur Einstellung der Brennweite von einer elektronischen Steuerung ansteuerbar. Eine derartige elektronische Steuerung kann bei einer medizinischen Stirnlampe mit Beobachtungseinrichtung bzw. bei einem Endoskop oder Exoskop beispielsweise dazu vorgesehen sein, eine Videokamera, eine Lichtquelle oder andere Funktionen anzusteuern. Dadurch, dass die Flüssiglinse durch die elektronische Steuerung ansteuerbar ist, wird beispielsweise eine Anpassung der Brennweite gemäß einer von einem Benutzer über eine Eingabeeinrichtung, etwa eine Sprachsteuerung, eingegebenen Vorgabe oder auch eine automatische Fokussierung durch die elektronische Steuerung und somit eine besonders einfache und sichere Bedienung ermöglicht. Da zur Einstellung der Beleuchtungsoptik keine Berührung der Beleuchtungsoptik notwendig ist, können außerdem Sterilitätsprobleme vermieden werden.

In dem Fall, dass die Flüssiglinse entlang einer optischen Achse der Linsenanordnung beweglich, insbesondere verschiebbar ist, kann auch eine Ansteuerung der Bewegung bzw. Verschiebung der Flüssiglinse, etwa durch Ansteuerung eines elektromotorischen Stellelements, durch die elektronische Steuerung vorgesehen sein. Hierdurch kann in besonders vorteilhafter Weise eine Kopplung der Bewegung der Flüssiglinse entlang der optischen Achse mit der Verstellung der Brennweite der Flüssiglinse realisiert werden, wodurch beispielsweise eine Anpassung eines Beleuchtungswinkels und eine Fokussierung der Beleuchtungsoptik in Abhängigkeit vom Arbeitsabstand ermöglicht wird. Insbesondere kann die elektronische Steuerung zur Erfassung des Sichtwinkels und zur automatischen Einstellung der Beleuchtungsoptik zur Anpassung des Beleuchtungswinkels an den Sichtwinkel ausgebildet sein. Dabei kann der Beleuchtungswinkel insbesondere geringfügig größer als der Sichtwinkel gewählt werden, um eine Verdunkelung im Randbereich des Sichtfelds der Beobachtungseinrichtung sicher zu vermeiden, die beispielsweise durch einen Versatz zwischen Beobachtungseinrichtung und Beleuchtungsoptik oder durch eine Randunschärfe des ausgeleuchteten Bereichs verursacht würde. Hierdurch wird eine besonders einfache und sichere Bedienung ermöglicht.

Bei einem erfindungsgemäßen Verfahren zum Erzeugen eines Beleuchtungslichtbündels mit veränderbarer Lichtverteilung wird eine Flüssiglinse mit veränderbarer Brennweite genutzt, um die Lichtverteilung des Beleuchtungslichtbündels zu verändern. Insbesondere kann durch eine Veränderung der Brennweite der Flüssiglinse eine Veränderung der Fokussierung des Beleuchtungslichtbündels erreicht werden. Eine Beleuchtungsoptik, die das Beleuchtungslichtbündel zum Ausleuchten eines Objektbereichs erzeugt, kann eine Linsenanordnung aufweisen, die die Flüssiglinse mit veränderbarer Brennweite umfasst und weitere optische Elemente umfassen kann. Hierdurch kann auf besonders einfache und wirksame Weise ein Beleuchtungslichtbündel mit veränderbarer Lichtverteilung erzeugt werden.

Insbesondere sind die oben beschriebene Beleuchtungsoptik und die oben beschriebene Beobachtungseinrichtung zur Durchführung des erfindungsgemäßen Verfahrens geeignet.

Weitere Aspekte ergeben sich aus der nachfolgenden Beschreibung eines bevorzugten Ausführungsbeispiels und der beigefügten Zeichnung. Es zeigen:
Fig. 1 eine Beleuchtungseinheit einer Stirnlampe gemäß einem ersten Ausführungsbeispiel der Erfindung in einem schematischen Längsschnitt;
Fig. 2 eine separate Beleuchtungseinheit für ein Endoskop oder ein Exoskop mit einer Beleuchtungsoptik gemäß einem zweiten Ausführungsbeispiel der Erfindung in einem schematischen Teilschnitt;
Fig. 3 ein Exoskop mit einer integrierten Beleuchtungseinheit in einer Seitenansicht;
Fig. 4 eine schematische Darstellung des Strahlengangs eines Ausführungsbeispiels einer erfindungsgemäßen Beleuchtungsoptik mit einem ersten Objektfelddurchmesser;
Fig. 5 eine schematische Darstellung des Strahlengangs des Ausführungsbeispiels der Fig. 4 mit einem zweiten Objektfelddurchmesser.

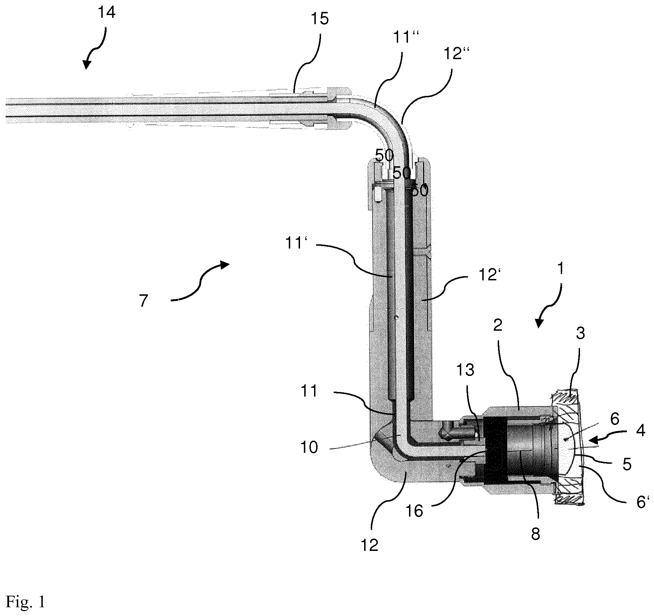

Wie in Fig. 1 schematisch dargestellt, umfasst eine Beleuchtungsoptik 1 gemäß einem Ausführungsbeispiel der Erfindung eine im Wesentlichen zylindrische Hülse 2, an deren Außenseite in ihrem distalen Endbereich ein manuell verstellbares Drehrad 3 angeordnet ist. Innerhalb des Drehrads 3 und innerhalb der Hülse 2 ist eine Flüssiglinse 4 angeordnet, die eine Grenzfläche 5 zwischen zwei flüssigen oder gelartigen optischen Medien 6, 6' aufweist, und die in dem dargestellten Beispiel als Sammellinse (entsprechend einer Konvexlinse) wirkt. Im dargestellten Ausführungsbeispiel stellt die Flüssiglinse 4 die einzige optisch wirksame Fläche der Beleuchtungsoptik 1 dar. Innerhalb der Hülse 2 können jedoch noch weitere optische Elemente, insbesondere weitere Linsen, angeordnet sein; in diesem Fall kann die Flüssiglinse 4 auch zur Wirkung als Zerstreuungslinse (entsprechend einer Konkavlinse) ausgebildet bzw. einstellbar sein.

Die Beleuchtungseinheit 7 einer medizinischen Stirnlampe umfasst ferner einen Lichtleiter 10, der beispielsweise aus einem Bündel von Lichtleitfasern besteht. Der Lichtleiter 10 ist von einer Hülle 11, 11', 11" umgeben und wird durch Führungselemente 12, 12', 12" in die Beleuchtungsoptik 1 geführt. Während das distale Ende des Lichtleiters durch eine Lichtleiterfassung 13 zentriert zur optischen Achse 8 der Beleuchtungsoptik 1 gehalten wird, läuft das proximale Ende des Lichtleiters in ein Lichtleitkabel 14 aus, das mit einer externen Lichtquelle verbindbar ist. Das Lichtleitkabel wird am Ansatz des Führungselements 12" durch einen Knickschutz 15 geschützt.

Zur Ausleuchtung eines Objektbereiches wird in einer in Fig. 1 nicht dargestellten externen Lichtquelle, beispielsweise einer Xenon-Lichtquelle, Licht in den Lichtleiter 10 eingekoppelt. Dieses Licht tritt an der distalen Endfläche 16 des Lichtleiters 10 aus diesem aus und wird durch die Flüssiglinse 4 auf einen Objektbereich projiziert. Die Flüssiglinse 4 ist in festem Abstand zur distalen Endfläche 16 des Lichtleiters 10 angeordnet. Durch Drehen des Drehrads 3 kann der Radius einer Fassung der Flüssiglinse 4, die die optischen Medien 6, 6' enthält, verändert werden. Hierdurch verändert sich die Krümmung der Grenzfläche 5 und damit die Brennweite der Flüssiglinse 4. Auf diese Weise kann die Fokussierung des von der Beleuchtungsoptik abgegebenen Lichtbündels derart eingestellt werden, dass Objektbereiche in unterschiedlichen Arbeitsabständen jeweils randscharf ausgeleuchtet werden können. Ein Lichtverlust durch Ausleuchten von nicht benötigten Bereichen eines Objekts kann hierdurch vermieden werden. Innerhalb der Hülse 2 können weitere optische Elemente, insbesondere zum Sammeln des aus der distalen Endfläche 16 des Lichtleiters 10 austretenden Lichts und damit zur Vermeidung eines Lichtverlusts sowie zur Verbesserung der Homogenität der Ausleuchtung innerhalb des Beleuchtungslichtkegels angeordnet sein.

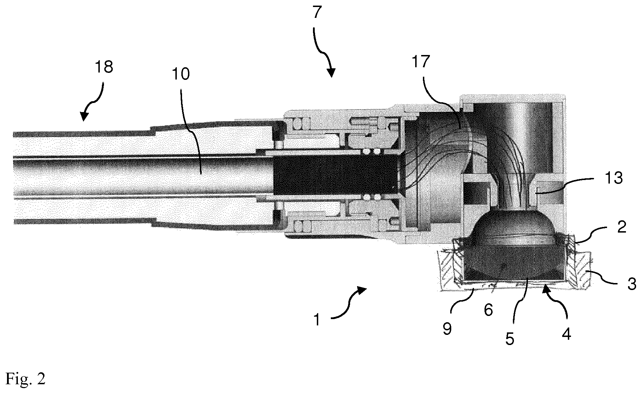

Fig. 2 zeigt eine weitere Ausführungsform einer Beleuchtungsoptik, wobei entsprechende Elemente mit den gleichen Bezugszeichen wie in Fig. 1 bezeichnet sind. Die Beleuchtungseinheit 1 umfasst eine Hülse 2, auf der ein Drehrad 3 drehbar angeordnet ist zur Verstellung der Brennweite einer fest innerhalb der Hülse 2 angeordneten Flüssiglinse 4. Die Hülse 2 weist außerdem ein Deckglas 9 auf, das dicht mit der Hülse 2 verbunden ist. Der Lichtleiter 10 umfasst ein Bündel von symbolisch dargestellten Lichtleitfasern 17, die in eine Lichtleiterfassung 13 münden, mit der sie in der Beleuchtungsoptik 1 gehalten sind. Die in Fig. 2 dargestellte Beleuchtungseinheit 7 ist insbesondere als separate Beleuchtungseinheit für ein Exoskop konzipiert und kann hierfür einen verstellbaren Schwanenhals 18 aufweisen. Die Beleuchtungseinheit 7 kann aber auch mit einem Exoskop oder einem Endoskop gekoppelt oder in ein solches integriert sein.

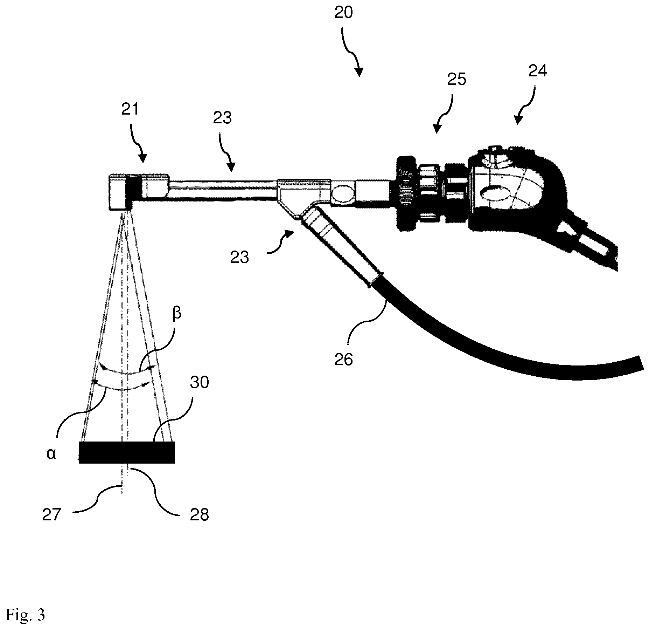

Ein Exoskop mit einer Beleuchtungseinheit ist in Fig. 3 dargestellt. Im distalen Endbereich 21 des Exoskops 20 ist eine gemäß einem weiteren Ausführungsbeispiel ausgebildete Beleuchtungsoptik integriert, die über ein Lichtleitkabel 22 und in einem Schaft 23 des Exoskops 20 aufgenommene Lichtleitfasern mit dem von einer externen Beleuchtungseinheit erzeugten Beleuchtungslicht versorgt wird. Die Beleuchtungsoptik erzeugt einen Lichtkegel mit einem Öffnungswinkels α, der auch als Beleuchtungswinkel bezeichnet wird.

Im distalen Endbereich 21 des Exoskops 20 ist ferner ein Objektiv einer Bildaufnahmeeinrichtung enthalten, das insbesondere als Zoomobjektiv ausgebildet sein kann. Das Exoskop 20 ermöglicht dadurch die Beobachtung eines Objekts unter einem veränderbaren Sichtwinkel β. Das von dem Objektiv aufgenommene Licht wird durch einen im Schaft 23 enthaltenen Bildweiterleiter, beispielsweise Stablinsen oder ein geordnetes Bündel von Lichtleitfasern, zu einer im Kopf 24 des Exoskops enthaltenen Videokamera weitergeleitet. Die Videokamera kann auch in miniaturisierter Form im distalen Endbereich 21 des Exoskops angeordnet sein. Der Kopf 24 des Exoskops ist über eine Kupplung 25 mit dem Schaft 23 verbunden. Auch das Lichtleitkabel 22 ist mit dem Schaft 23 über eine Kupplung 26 lösbar verbunden.

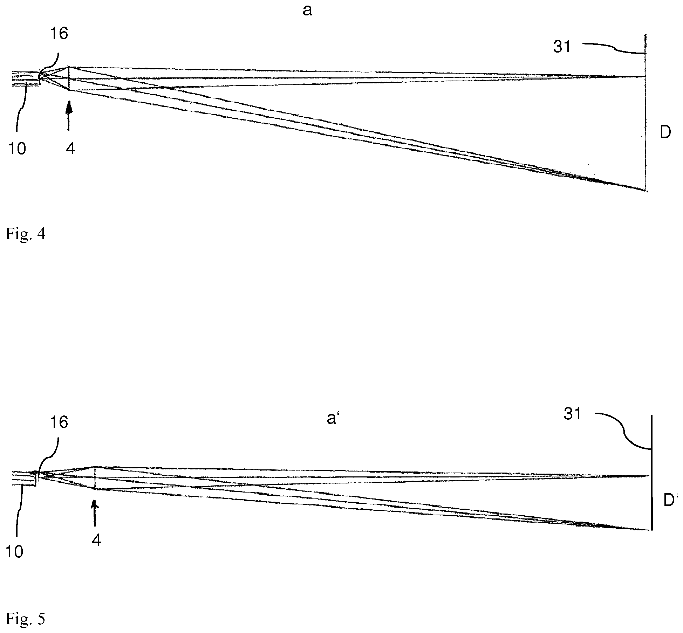

Wie in Fig. 3 schematisch dargestellt, ist der Beleuchtungswinkel α derart gewählt, dass er im Wesentlichen mit einem Sichtwinkel β der Videokamera, der durch das Zoomobjektiv veränderbar ist, übereinstimmt. Der geringfügige Versatz von beispielsweise wenigen Millimetern zwischen der optischen Achse 27 der Beleuchtungsoptik und der optischen Achse 28 der Beobachtungsoptik ist für die Ausleuchtung des Objektbereichs 30 insbesondere dann unerheblich, wenn dieser einen Abstand vom distalen Endbereich 21 des Exoskops aufweist, der wesentlich größer als der Versatz ist, also beispielsweise einen Abstand von einigen Zentimetern. Der Beleuchtungswinkel α kann insbesondere geringfügig größer als der Sichtwinkel β gewählt werden, so dass auf jeden Fall bei allen gewünschten Arbeitsabständen eine vollständige Ausleuchtung des Objektbereichs 30 erzielt wird. Durch axiale Verschiebung der Flüssiglinse kann der Beleuchtungswinkel α derart geändert werden, dass er stets an den mit Hilfe des Zoomobjektivs veränderbaren Sichtwinkel β der Kamera angepasst ist. Durch eine Veränderung der Brennweite der Flüssiglinse wird eine Fokussierung erreicht, so dass die Ausleuchtung des Objektbereichs 30 mit einem scharf definierten Rand erfolgt. Hierdurch wird bei jeder möglichen Vergrößerung, die durch Einstellung des Zoomobjektivs gewählt wird, stets eine optimale randscharfe Ausleuchtung des Sichtfelds erreicht. Durch eine nicht dargestellte elektronische Steuerung kann der Sichtwinkel β der Beobachtungsoptik sowie ggf. ein Abstand zwischen dem distalen Endbereich 21 des Exoskops und dem Objektbereich erfasst werden und die Verschiebung der Flüssiglinse sowie die Einstellung der Brennweite der Flüssiglinse automatisch derart gesteuert werden, dass stets das Sichtfeld der Beobachtungsoptik vollständig ausgeleuchtet ist und ein möglichst geringer Anteil des Beleuchtungslichts Bereiche außerhalb des Sichtfelds beleuchtet und das beleuchtete Objektfeld stets scharf begrenzt ist. Der Strahlengang der Beleuchtungsoptik wird anhand von Fig. 4 und 5 beispielhaft erläutert.

Wie in Fig. 4 und 5 in schematischer Darstellung gezeigt, wird das aus einer distalen Endfläche 16 eines Lichtleiters 10 austretende Beleuchtungslicht von einer Flüssiglinse 4 in eine Objektebene 31 abgebildet, so dass in der Objektebene 31 ein Bild der Endfläche 16 des Lichtleiters entsteht, das einen Bildradius R aufweist. In der in Fig. 4 gezeigten Anordnung ist die Flüssiglinse 4 auf eine relativ kurze Brennweite eingestellt, beispielsweise auf eine Brennweite von 12,5 mm, so dass in einem Abstand a = 250 mm der Objektebene 30 von der Flüssiglinse 4 eine ausgeleuchtete Fläche mit einem Durchmesser D = 10 mm entsteht.

Gemäß Fig. 5 kann die Flüssiglinse 4 auf eine größere Brennweite von beispielsweise 25 mm eingestellt werden. In diesem Fall entsteht in der Objektebene 31, die weiterhin einen Abstand a' = a = 250 mm von der Flüssiglinse 4 aufweisen kann, ein Bild der Endfläche des Lichtleiters mit einem Durchmesser D' = 5 mm Der Abstand der Flüssiglinse 4 von der Endfläche 16 des Bildleiters 10 ist entsprechend der Vergrößerung der Brennweite der Flüssigkeitslinse 4 in Fig. 5 gegenüber der Anordnung in Fig. 4 von ca. 12,5 mm auf ca. 25 mm vergrößert.

Durch eine Anpassung der Brennweite und eine axiale Verschiebung der Flüssiglinse entsprechend einer veränderten Brennweite der Kamera bzw. der Beobachtungsoptik kann der Durchmesser des ausgeleuchteten Objektbereichs, der hier dem Durchmesser D bzw. D' des Bilds der Lichtleiterendfläche 16 entspricht, angepasst werden. Der Objektbereich kann dabei umso heller beleuchtet werden, je kleiner der Durchmesser gewählt wird. So kann etwa, wenn keine Lichtverluste auftreten, die Flächenhelligkeit des ausgeleuchteten Bereichs in Fig. 5 viermal so hoch sein wie in Fig. 4. Durch Kopplung eines elektrischen Signals des Zoomobjektivs der Kamera mit einer elektrisch ansteuerbaren Flüssiglinse kann man somit bei jeder gewählten Vergrößerung ein homogenes, gleich stark ausgeleuchtetes Bild erhalten.

In Fig. 4 und 5 ist der Einfachheit halber eine Fokussierung auf die Objektebene 31 dargestellt; eine geringfügig defokussierte Abbildung kann jedoch zur Verbesserung der Homogenität der Ausleuchtung des Objektbereichs vorteilhaft sein.

Der Übersichtlichkeit halber sind nicht in allen Figuren alle Bezugszeichen dargestellt. Zu einer Figur nicht erläuterte Bezugszeichen haben die gleiche Bedeutung wie in den übrigen Figuren.

### Bezugszeichenliste

- 1: Beleuchtungsoptik
- 2: Hülse
- 3: Drehrad
- 4: Flüssiglinse
- 5: Grenzfläche
- 6, 6': Optisches Medium
- 7: Beleuchtungseinheit
- 8: Optische Achse
- 9: Deckglas
- 10: Lichtleiter
- 11, 11', 11": Hülle
- 12, 12 12": Führungselement
- 13: Lichtleiterfassung
- 14: Lichtleitkabel
- 15: Knickschutz
- 16: Distale Endfläche
- 17: Lichtleitfaser
- 18: Schwanenhals
- 20: Exoskop
- 21: Distaler Endbereich
- 22: Lichtkabel
- 23: Schaft
- 24: Kopf
- 25: Kupplung
- 26: Kupplung
- 30: Objektbereich
- 31: Objektebene

## Patentansprüche

1. Beleuchtungsoptik für eine medizinische Stirnlampe, ein Endoskop oder ein Exoskop (20) zum Erzeugen eines Beleuchtungslichtbündels mit veränderbarer Lichtverteilung, wobei die Beleuchtungsoptik (1) eine Flüssiglinse (4) mit veränderbarer Brennweite zur Veränderung einer Fokussierung des Beleuchtungslichtbündels aufweist, und eine im Wesentlichen zylindrische Hülse (2) umfasst, an deren Außenseite in ihrem distalen Endbereich ein manuell verstellbares Drehrad (3) angeordnet ist,
wobei eine Einstellung der Brennweite der Flüssiglinse (4) durch mechanische Mittel erfolgt und ein mechanisches Bedienelement direkt oder über ein Getriebe auf die Flüssiglinse (4) zur Veränderung der Brennweite einwirkt,
wobei das mechanische Bedienelement das Drehrad (3) ist, und die Flüssiglinse (4) eine Grenzfläche (5) zwischen zwei flüssigen oder gelartigen optischen Medien (6, 6') aufweist,
**dadurch gekennzeichnet, dass**
innerhalb des Drehrads (3) und innerhalb der Hülse (2) die Flüssiglinse (4) angeordnet ist.

2. Beleuchtungsoptik nach einem der vorhergehenden Ansprüche, **dadurch gekennzeichnet, dass** die Beleuchtungsoptik (1) zum randscharfen Ausleuchten eines Objektbereichs (30) in unterschiedlichen Arbeitsabständen (a, a') durch Einstellung unterschiedlicher Brennweiten der Flüssiglinse (4) ausgebildet ist.

3. Beleuchtungsoptik nach dem vorhergehenden Anspruch, **dadurch gekennzeichnet, dass** die Beleuchtungsoptik (1) zur Abbildung einer Lichtaustrittsebene auf den Objektbereich (30) ausgebildet ist.

4. Beleuchtungsoptik nach einem der vorhergehenden Ansprüche, **dadurch gekennzeichnet, dass** die Beleuchtungsoptik (1) zum Verändern eines Öffnungswinkels des Beleuchtungslichtbündels ausgebildet ist.

5. Beleuchtungsoptik nach dem vorhergehenden Anspruch, **dadurch gekennzeichnet, dass** die Flüssiglinse (4) entlang einer optischen Achse (7) der Beleuchtungsoptik (1) verschiebbar ist.

6. Beleuchtungsoptik nach dem vorhergehenden Anspruch, **dadurch gekennzeichnet, dass** eine Veränderung der Brennweite und eine Verschiebung der Flüssiglinse (4) miteinander gekoppelt sind.

7. Beobachtungseinrichtung mit einer Beleuchtungsoptik (1) gemäß einem der vorhergehenden Ansprüche.

8. Beobachtungseinrichtung nach dem vorhergehenden Anspruch, **dadurch gekennzeichnet, dass** die Beobachtungseinrichtung eine Beobachtungsoptik mit einem veränderbaren Sichtwinkel (β) aufweist und dass die Beleuchtungsoptik (1) zur Anpassung eines Beleuchtungswinkels (α) an den Sichtwinkel (β) ausgebildet ist.

9. Beobachtungseinrichtung nach einem der Ansprüche 7 oder 8, **dadurch gekennzeichnet dass**, die Beobachtungseinrichtung eine medizinische Stirnlampe, ein Endoskop oder ein Exoskop (20) ist.

## Claims

1. Illumination optical unit for a medical headlamp, an endoscope or an exoscope (20), for producing an illumination light beam with a changeable light distribution, wherein the illumination optical unit (1) has a liquid lens (4) with a changeable focal length for changing a focus of the illumination light beam and comprises a substantially cylindrical sleeve (2), at the outer side of which a manually adjustable rotating wheel (3) is arranged in the distal end region thereof,
wherein the focal length of the liquid lens (4) is set by mechanical means and a mechanical operating element acts directly, or via a transmission, on the liquid lens (4) for the purposes of changing the focal length,
wherein the mechanical operating element is the rotating wheel (3) and the liquid lens (4) has an interface (5) between two liquid or gel-like optical media (6, 6'),
**characterized in that**
the liquid lens (4) is arranged within the rotating wheel (3) and within the sleeve (2).

2. Illumination optical unit according to one of the preceding claims, **characterized in that** the illumination optical unit (1) is embodied to illuminate an object region (30) in a sharply contoured manner at different working distances (a, a') by setting different focal lengths of the liquid lens (4).

3. Illumination optical unit according to the preceding claim, **characterized in that** the illumination optical unit (1) is embodied to image a light-exit plane on the object region (30).

4. Illumination optical unit according to one of the preceding claims, **characterized in that** the illumination optical unit (1) is embodied to change an aperture angle of the illumination light beam.

5. Illumination optical unit according to the preceding claim, **characterized in that** the liquid lens (4) is displaceable along an optical axis (7) of the illumination optical unit (1).

6. Illumination optical unit according to the preceding claim, **characterized in that** a change in the focal length and a displacement of the liquid lens (4) are coupled to one another.

7. Observation device comprising an illumination optical unit (1) according to one of the preceding claims.

8. Observation device according to the preceding claim, **characterized in that** the observation device has an observation optical unit with a changeable viewing angle (β) and **in that** the illumination optical unit (1) is embodied to match an illumination angle (α) with the viewing angle (β).

9. Observation device according to either of Claims 7 and 8, **characterized in that** the observation device is a medical headlamp, an endoscope or an exoscope (20).

## Revendications

1. Optique d'éclairement destinée à une lampe frontale médicale, un endoscope ou un exoscope (20), pour générer un faisceau lumineux d'éclairement ayant une distribution lumineuse modifiable, dans lequel l'optique d'éclairement (1) comporte un objectif liquide (4) ayant une distance focale modifiable pour modifier une focalisation du faisceau lumineux d'éclairement, et comprend un manchon sensiblement cylindrique (2) sur la face extérieure duquel est disposé, dans sa région d'extrémité distale, un sélecteur rotatif (3) réglable manuellement,
dans lequel un réglage de la distance focale de l'objectif liquide (4) est effectué par des moyens mécaniques et un élément mécanique de commande agit directement ou par l'intermédiaire d'un engrenage sur l'objectif liquide (4) pour modifier la distance focale,
dans lequel l'élément mécanique de commande est le sélecteur rotatif (3), et l'objectif liquide (4) comporte une surface limite (5) entre deux milieux optiques (6, 6') liquides ou de type gel,
**caractérisée en ce que** l'objectif liquide (4) est disposé à l'intérieur du sélecteur rotatif (3) et à l'intérieur du manchon (2).

2. Optique d'éclairement selon l'une quelconque des revendications précédentes, **caractérisée en ce que** l'optique d'éclairement (1) est réalisée de manière à éclairer de manière précise suivant les bords une zone objet (30) à différentes distances de travail (a, a') par réglage de différentes distances focales de l'objectif liquide (4).

3. Optique d'éclairement selon la revendication précédente, **caractérisée en ce que** l'optique d'éclairement (1) est réalisée pour la formation d'image d'un plan de sortie de lumière sur la région objet (30).

4. Optique d'éclairement selon l'une quelconque des revendications précédentes, **caractérisée en ce que** l'optique d'éclairement (1) est réalisée de manière à modifier un angle d'ouverture du faisceau lumineux d'éclairement.

5. Optique d'éclairement selon la revendication précédente, **caractérisée en ce que** l'objectif liquide (4) peut être déplacé le long d'un axe optique (7) de l'optique d'éclairement (1).

6. Optique d'éclairement selon la revendication précédente, **caractérisée en ce qu'**une modification de la distance focale et un décalage de l'objectif liquide (4) sont couplés l'un à l'autre.

7. Dispositif d'observation comportant une optique d'éclairement (1) selon l'une quelconque des revendications précédentes.

8. Dispositif d'observation selon la revendication précédente, **caractérisé en ce que** le dispositif d'observation comporte une optique d'observation ayant un angle de vue modifiable (β) et **en ce que** l'optique d'éclairement (1) est réalisée de manière à adapter un angle d'éclairement (α) à l'angle de vue (β).

9. Dispositif d'observation selon l'une quelconque des revendications 7 ou 8, **caractérisé en ce que** le dispositif d'observation est une lampe frontale médicale, un endoscope ou un exoscope (20).
